# EUROPEAN PATENT APPLICATION

(11) **EP 4 458 367 A1**
(43) Date of publication of application: **06.11.2024**
(21) Application number: 22915020.6
(22) Date of filing: 29.12.2022
(51) Int. Cl.: A61K 38/08, A61P 17/00

(54) **APPLICATION OF POLYPEPTIDE IN PREPARATION OF PRODUCT FOR PREVENTING OR TREATING SKIN INJURY DISEASES**

(30) Priority: 29.12.2021 CN 202111643000
(71) Applicant: Sichuan Gooddoctor Panxi Pharmaceutical Co. Ltd, Sichuan 615000 (CN)
(72) Inventor: GENG, Funeng, Autonomous Prefecture Sichuan 615000 (CN); GENG, Yuefei, Autonomous Prefecture Sichuan 615000 (CN); LIU, Bin, Autonomous Prefecture Sichuan 615000 (CN); JIANG, Shunri, Autonomous Prefecture Sichuan 615000 (CN)
(74) Representative: Mewburn Ellis LLP
(86) International application number: PCT/CN2022/143243
(87) International publication number: WO 2023/125770

(57) **Abstract**

Provided is the use of a polypeptide in the preparation of a product for preventing or treating skin injury diseases. The polypeptide provided herein not only has obvious wound-healing effect on skin ulcers and injuries, especially chronic refractory wounds on a body surface and acute and/or chronic skin diseases, but also can be absorbed by the skin much faster and much better due to its short chain of the peptide so as to have good stability in vivo and in vitro, and have a significant pro-proliferation effect on human immortalized keratinocytes, human microvascular endothelial cells, fibroblasts, glial cells, and tissue regeneration and angiogenesis. Therefore, the polypeptide provided herein is suitable for preparing a product for preventing or treating skin injury diseases and can produce positive therapeutic and preventive effects.

## Description

### Field of the Invention

Provided is the use of a polypeptide in the preparation of a product for preventing or treating skin injury diseases. The polypeptide provided herein is able to significantly prevent and/or treat skin injury diseases, especially chronic refractory wounds on a body surface, and acute and/or chronic skin diseases.

### Background of the Invention

Human skin is composed of epidermis, dermis, and subcutaneous tissues, and contains accessory organs (sweat glands, sebaceous glands), blood vessels, lymphatic vessels, nerves, muscles, etc. The skin is the outermost organ of the human body, and it is also the largest organ in the human body. It also has the most frequent and closest contact with the outside world. Skin covers the surface of the human body. In addition to protecting the body, regulating body temperature, and resisting external aggression, the skin also has sensory functions. The skin plays an important role in protecting human health, so it is said that the skin is the first line of defense of the human body. Since the skin covers the surface of the human body, it is vulnerable to external damage, chemical stimulation, microbial infection and the like. Skin injuries or lesions refer to abnormalities that can be seen or touched on the skin and mucous membranes. Patients with skin injuries would suffer from the skin injuries both psychologically and physically.

In recent years, among the diseases caused by skin injuries, chronic refractory wound diseases on a body surface have received greater attention. Chronic refractory wounds on a body surface, also known as refractory ulcers, are common refractory diseases. Local tissue defects, liquefaction, infection, and necrosis caused by various reasons lead to impaired proliferation of endothelial progenitor cells, resulting in angiogenesis disorders, insufficient wound perfusion, metabolic disorders, and delayed epithelial cell formation, which disrupts the normal healing process of wounds, resulting in the formation of chronic ulcers.

Chronic refractory wounds on the body surface are common complications of diabetes, peripheral vascular diseases, radiotherapy and the like. The International Wound Healing Society defines "chronic refractory wounds on the body surface" as: a wound that can not achieve an anatomically and functionally complete state through a normal, orderly and timely healing process. Clinically, "chronic refractory wounds on a body surface" mostly refer to wounds formed by various reasons, which fail to heal after not less than one month of regular treatment, and have no obvious tendency to heal. Wound healing goes through three stages: (1) inflammation stage; (21) proliferation stage; and (3) maturation and remodeling stage. Chronic wounds hinder the proliferation of vascular endothelial cells and fibroblasts and the deposition of collagen matrix due to the prolonged inflammatory phase.

Common chronic refractory wounds include diabetic foot ulcers, pressure ulcers, vascular ulcers, neurotrophic ulcers, infectious ulcers, traumatic ulcers, autoimmune ulcers, cancerous ulcers, and radiation ulcers, and so on. The incidence of the chronic refractory wounds increases with age, and the pathogenesis thereof is complex with a long course of disease.

Diabetic foot ulcers are mainly caused by neurological and vascular lesions caused by diabetes, resulting in ischemic necrosis of skin tissues or infection of skin tissues, or damage caused by pressure on bearing points to result in incomplete skin, and exposed subcutaneous fat, muscle tissue, and even bones, so as to form ulcers. The injuries caused by diabetic foot ulcers can lead to mild injuries, i.e., skin itching, no sweat, dryness, pigmentation, slight pain in the feet, insensitivity, intermittent claudication, joint deformation, and skin surface ulcers; further develop to moderate injuries, i.e., a relatively deep ulcers combined with infection; severe injuries, i.e., bone involvement resulting in fractures and bone necrosis; and more severe injuries requiring amputation. Diabetic foot ulcer is one of the important causes of disability and even death of diabetic patients, which not only causes pain to patients, but also adds a huge economic burden to them. At present, diabetic foot ulcers are mostly treated by controlling the treatment of the underlying disease, but local ulcers are prone to infection during the treatment process, resulting in serious consequences because the treatment process is relatively slow.

Pressure ulcers are the tissue damage and necrosis resulting from local skin ischemia, hypoxia, and nutritional deficiency of normal skin due to the compression of local tissues of the body and the disturbance of blood circulation. Pressure ulcers often occur in bony protrusions of bedridden elderly patients, such as sacrum, occiput, and heel. These locations have less soft tissue and weak pressure resistance, and ulcers can occur when the body position is fixed and the body is pressed for a long time. Pressure sores not only bring pain to patients and delay the recovery of the disease, but also are life-threatening due to secondary infection and septicemia in severe cases.

Vascular ulcers are lower extremity ulcers caused by varicose veins and vasculitis of the lower extremities. They are more common in the distal calf and ankle and are a complication of the late stage of chronic insufficiency of lower extremities. The severely obstructed venous return, the increase of local venous pressure and edemas lead to oxygen diffusion barrier and skin nutrition deficiency. The patients have a long-term history of underlying venous diseases. The wound is generally single and superficial with dark wound base, rough skin around the wound and obvious pigmentation. The local skin temperature is very low. The therapeutic effect of routine wound dressing change is very poor, and the therapeutic effect is extremely unreliable even with split skin grafting.

Current studies have found that skin wound healing is a complex process involving various factors such as keratinocytes, fibroblasts, vascular endothelial cells, inflammatory cells, extracellular matrix, cytokines, and growth factors, which are highly coordinated and mutually regulated. At present, drugs commonly used in the prevention or treatment of skin injury diseases include recombinant human epidermal growth factors, etc. Although the recombinant human epidermal growth factors have been found to have certain preventive and therapeutic effects, they still have disadvantages such as slow absorption, long treatment cycle, and ineffective therapeutic effect. Especially for chronic refractory wounds on a body surface and acute and/or chronic skin diseases, the therapeutic effect is minimal.

### Summary of the Invention

In order to overcome the deficiencies and defects of the prior art, the object of the present invention is to provide the use of a polypeptide in the preparation of a product for preventing or treating skin injury diseases.

In the first aspect, provided is the use of a polypeptide in the preparation of a product for preventing or treating skin injury diseases, the polypeptide is Pro-Ala-Ala-Glu-Pro-Val-Pro-Leu or a physiologically compatible salt thereof; or provided is a method for preventing or treating skin injury diseases, said method comprising locally administering a product comprising Pro-Ala-Ala-Glu-Pro-Val-Pro-Leu or a physiologically compatible salt thereof to a skin lesion site; or provided is a product comprising Pro-Ala-Ala-Glu-Pro-Val-Pro-Leu or a physiologically compatible salt thereof for use in the prevention or treatment of skin injury diseases.

Further, the skin injury diseases comprise wounds, ulcers, dermatitis, eczema, urticaria, polymorphic light rash, herpes, acne, impetigo, chloasma, vitiligo, lupus erythematosus, dermatomyositis, scleroderma, folliculitis, scabies, onychomycosis, chickenpox, roseola infantilis, warts, carbuncles, boils or ringworms.

Further, the dermatitis comprises atopic dermatitis, contact dermatitis, neurodermatitis, seborrheic dermatitis, hormone-dependent dermatitis or stasis dermatitis.

Further, the wounds comprise chronic refractory wounds on a body surface.

Further, the chronic refractory wounds on a body surface comprise diabetic foot ulcers, pressure ulcers, vascular ulcers and infectious ulcers.

Further, the product comprises medicines, skin care products or cosmetics.

Further, the product is a topical preparation.

Further, the dosage form of the topical preparation comprises a solution, emulsion, gel, cream, spray, mask or dressing.

Further, the polypeptide provided herein can repair or heal the wounds by promoting the proliferation of human immortalized keratinocytes, human microvascular endothelial cells, fibroblasts, glial cells, and tissue regeneration and/or angiogenesis.

Further, a physiologically compatible salt refers to a salt form that is physiologically compatible (i.e., pharmacologically acceptable) and substantially non-toxic to an individual to whom the compound provided herein is to be administered. Physiologically compatible salts of the compound provided herein include conventional and stoichiometric acid addition salts or base addition salts formed from suitable, non-toxic organic or inorganic acids or inorganic bases.

The use of the polypeptide provided herein has the following beneficial effects in preparing a product for preventing or treating skin injury diseases, especially chronic refractory wounds on a body surface:

Compared with the recombinant human epidermal growth factors commonly used in the treatment of skin injury diseases in the art, the polypeptide provided herein not only has obvious wound-healing effect on skin ulcers and injuries, but also can be absorbed by the skin much faster and much better due to its short chain of the peptide so as to have good stability in vivo and in vitro, and have a significant pro-proliferation effect on human immortalized keratinocytes, human microvascular endothelial cells, fibroblasts, glial cells, and tissue regeneration and angiogenesis. Therefore, the polypeptide provided herein is able to significantly prevent and/or treat acute and/or chronic skin diseases and chronic refractory wounds on a body surface especially (diabetic foot ulcers, pressure ulcers, vascular ulcers and infectious ulcers). The polypeptide provided herein is suitable for preparing a product for preventing or treating skin injury diseases, and can induce the dedifferentiation of cells near the injury, and the dedifferentiated cells regain the stem cell morphology and cell division potential, form new units to heal the damaged wound, and can produce positive therapeutic and preventive effects.

### Brief Description of the Drawings

Figure 1 shows the proliferation-promoting effect of the polypeptide on the HaCaT cell line;
Figure 2 shows the proliferation-promoting effect of the polypeptide on the HMEC-1 cell line;
Figure 3 shows the proliferation-promoting effect of the polypeptide on the Balb-3T3 cell line;
Figure 4 shows the proliferation-promoting effect of the polypeptide on the RSC96 cell line;
Figure 5 shows the effect of the polypeptide on zebrafish caudal fin regeneration;
Figure 6 shows the effect of the polypeptide on the area of regenerated blood vessels in zebrafish with microvessel deficiency;
Figure 7 shows the effect of the polypeptide on the number of regenerated blood vessel branches in zebrafish with microvessel deficiency;
Figure 8 shows the effect of the polypeptide on the formation rate of new granulation tissues in STZ-induced diabetic rats on the 3rd day after injury.

### Detailed Description of the Invention

The following is a description of the present invention in conjunction with specific trials and is not a limitation on the scope of protection of the present invention.

### Example 1: Chemical synthesis of the polypeptide

The polypeptide was synthesized by a conventional solid-phase synthesis method via multiple cyclic processes of resin swelling, substitution, deprotection, washing, amino acid dissolution, amino acid activation and condensation processes, washing, and further deprotection, and finally cleavage and side chain deprotection.

Abbreviations: HBTU represents Benzotriazole-*N*,*N*,*N*',*N*'-tetramethyluronium hexafluorophosphate; Methanol represents methanol; Tert-Butyl methyl ether represents Tert-Butyl methyl ether; Ethanol represents ethanol; AA represents amino acid; Cl-2-Cl-Resin represents 2-chlorotrityl chloride resin; Fmoc-Aa(n) represents an amino acid with 9-fluorenylmethoxycarbonyl; DIPEA represents N,N-diisopropylethylamine; DCM represents dichloromethane; PIP represents piperidine; DMF represents N,N-dimethylformamide; HOBt represents 1-hydroxybenzotriazole; DIC represents N,N'-diisopropyl carbodiimide; TFA represents trifluoroacetic acid; and TIPS represents triisopropylsilane.

The polypeptide was synthesized and purified as follows:

### Step 1. Preparation of fully protected peptide resin

(1) Resin swelling: 2.0192 g of 2-Chlorotrityl Chloride Resin (S = 0.73 mmol/g) was weighed, added to a synthesis tube with a sieve plate, swelled with 40 ml of dichloromethane for 30 min, and subjected to suction filtration to remove dichloromethane.
(2) Preparation of Fmoc-Asp(OtBu)-Resin: Based on a molar ratio of 1 : 1.5 : 1.65 of resin to Fmoc-Asp(OtBu)-OH to DIPEA, Fmoc-Asp(OtBu)-OH and DIPEA were respectively weighed, dissolved in 20 ml of dichloromethane and added to the synthesis tube. Bubbling with N₂ and shaking were performed at room temperature for 1-3 hours, and 2 ml of methanol was directly added to the reaction solution, followed by blocking for 30 min. It was then washed 4 times separately with dimethylformamide, 25 ml each time, and the resin was dried by draining.
(3) Removal of Fmoc protecting group: 20 ml of a 20% piperidine-DMF (v/v) solution was added to the reactor, the reaction was bubbled with N₂ for 20 min, and draining was performed; and it was then washed with dimethylformamide 6 times, 25 ml each time, 3 min each time, and after draining, the results of Fmoc removal were detected by the ninhydrin method.
(4) Amino acid pre-activation: 4.38 mmol of Fmoc-protected amino acid, 5.26 mmol of HOBt, 4.60 mmol of DIC were added to a 250 ml round bottom flask, dissolved in 20 ml of 1 : 1 DCM-DMF (v/v), and preactivated in an ice bath at -5°C to 0°C under stirring for 30-60 min.
(5) Amino acid connection: the activated protected amino acid solution was poured into the reactor, and an appropriate amount of DCM was supplemented to clean the tools. After the reaction was bubbled with N₂ at room temperature for 1-3 hours, the ninhydrin method was used to detect whether the amino acid connection was complete and if so, draining was performed. The resin was washed with dimethylformamide 4 times, 25ml each time, 3 min each time, and draining was performed. The amounts of each amino acid and condensing agent and the specific reaction time were shown in Table 1.
(6) After the condensation of the first amino acid was complete, steps (3) to (5) were repeated to extend the peptide chain according to the amino acid sequence until the coupling of the last amino acid was completed.
(7) The resin peptide was washed with dichloromethane 6 times, 25 ml each time, 3 min each time, and draining was performed.

**Table 1 Amounts of amino acids and condensing agent**

| Amino acid name | AA/eq | Amino acid amount/g | HOBt/g | DIPEA/g | DIC/g |
|---|---|---|---|---|---|
| Fmoc-L-Leu-OH | 4.38 | 1.55 | 0.71 | 0.57 | 0.58 |
| Fmoc-L-Pro-OH | 4.38 | 1.48 | 0.71 | 0.57 | 1.16 |
| Fmoc-L-Val-OH | 4.38 | 1.49 | 0.71 | 0.57 | 0.58 |
| Fmoc-L-Pro-OH | 4.38 | 1.48 | 0.71 | 0.57 | 1.16 |
| Fmoc-L-Glu(OtBu)-OH·H₂O | 4.38 | 1.94 | 0.71 | 0.57 | 1.16 |
| Fmoc-L-Ala-OH H₂O | 4.38 | 1.44 | 0.71 | 0.57 | 1.16 |
| Fmoc-L-Ala-OH H₂O | 4.38 | 1.44 | 0.71 | 0.57 | 1.16 |
| Fmoc-L-Pro-OH | 4.38 | 1.48 | 0.71 | 0.57 | 1.16 |

### Step 2. Cleavage and deprotection

(1) 50 ml of a cleaving agent (TFA : TIPS : H₂O = 95 : 2.5 : 2.5, v/v) was added to the synthesis tube in step 1, and the reaction was bubbled with N₂ for 1.5-3 hours.
(2) After the cleavage reaction was complete, the cleaving agent was suction-filtered into a 250 ml round bottom flask. After vacuum concentration to one third of the original volume of the cleaving agent, 10 folds of the existing volume of methyl tert-butyl ether was added, and the mixture was stirred for 30 min. The resulting mixed solvent was filtered and washed three times separately with 30 ml of methyl tert-butyl ether, and the resulting crude peptide product was put into a sand core funnel and dried with N₂ in a fume hood, so that the solvent was volatilized until the crude peptide became powder.

### Step 3. Purification (salt exchange) and freeze-drying

Using the following chromatographic parameter condition A, the crude peptide obtained in step 2 was purified by HPLC. Specifically, the crude peptide obtained in step 2 was dissolved with water and/or acetonitrile, and filtered by a 0.45 µm filter membrane; sample injection was performed; gradient elution was performed with an acetonitrile-water mobile phase; a peptide eluate of interest was collected; and finally, rotary evaporation concentration was performed.

Chromatographic parameter condition A:
Chromatographic column: YMC-Actus Triart C18 30*250 mm;
Eluent A: 0.1% (v/v) TFA/H₂O;
Eluent B: acetonitrile;
Flow rate: 25 ml/min;
Ultraviolet detection wavelength: 220 nm;

**Table 2 Gradient elution conditions**

| Time, min | Eluent A (%) | Eluent B (%) |
|---|---|---|
| 0 | 90 | 10 |
| 30 | 75 | 25 |

Next, the product obtained in the previous step was subjected to salt exchange by HPLC method using the following chromatographic parameter condition B. Specifically, the chromatographic column was equilibrated with 95% A1 + 5% B; sample injection was then performed; next, the chromatographic column was equilibrated with 95% A2 + 5% B; gradient elution was performed with A1 and B; the peptide eluate of interest was collected; and finally, rotary evaporation concentration and freeze-drying were performed to obtain the polypeptide. The structure of the polypeptide was confirmed by MS and ¹H-NMR.

Chromatographic parameter condition B:
Chromatographic column: YMC-Actus Triart C18 30*250 mm
Eluent A1: 0.1 M acetic acid
Eluent A2: 0.025 M acetic acid + 0.1 M ammonium acetate
Eluent B: acetonitrile
Flow rate: 25 ml/min
Ultraviolet detection wavelength: 220 nm

**Table 3 Gradient elution conditions**

| Time, min | Eluent A1 (%) | Eluent B (%) |
|---|---|---|
| 0 | 95 | 5 |
| 5 | 95 | 5 |
| 35 | 70 | 30 |

### The polypeptide has the following ¹H-NMR data:

¹H NMR (600 MHz, DMSO) δ 8.25 (s, 1H), 8.09 (d, J = 7.5 Hz, 1H), 7.94 (d, J = 7.6 Hz, 1H), 7.89 (d, J = 8.3 Hz, 2H), 4.53 - 4.46 (m, 1H), 4.39 (dd, J = 8.3, 4.2 Hz, 1H), 4.34 (dd, J = 8.4, 3.8 Hz, 1H), 4.31 - 4.19 (m, 3H), 4.13 (dd, J = 15.1, 7.7 Hz, 1H), 3.71 - 3.49 (m, 5H), 2.94 - 2.77 (m, 2H), 2.33 - 2.20 (m, 2H), 2.06 - 1.77 (m, 13H, AcOH), 1.77 - 1.56 (m, 6H), 1.46 (t, J = 7.3 Hz, 2H), 1.25 - 1.11 (m, 6H), 0.95 - 0.76 (m, 12H).

**MS of the polypeptide:** 793.4, 397.3(double charge).

**The amino acid sequence of the polypeptide:** Pro-Ala-Ala-Glu-Pro-Val-Pro-Leu.

The following substances are used in the examples of this application:
The polypeptide, unless otherwise indicated, is the polypeptides having the amino acid sequence Pro-Ala-Ala-Glu-Pro-Val-Pro-Leu.

GeneTime (the trade name) has the common name: Recombinant Human Epidermal Growth Factor Derivative for External Use, Liquid; and is manufactured by Shenzhen Watsin Genetech Ltd. It contains the active ingredient recombinant human epidermal growth factor (rhEGF), with 10% glycerin and 1.0% mannitol as protective agents, and the rhEGF in GeneTime has the function of promoting the synthesis of DNA, RNA and hydroxyproline during the process of healing skin and mucosal wounds, thereby accelerating the generation of the wound granulation tissue and the proliferation of epithelial cells so as to shorten the healing time of the wound surface.

Kangfuxin Ye was manufactured by SICHUAN GOODDOCTOR PANXI PHARMACEUTICAL CO., LTD. and is a solution of Periplaneta americana dry worm body extract and is used for Wounds of gold sores, trauma, ulcers, fistulas, burns, scalds, and bedsores.

### Example 2 The effect of the polypeptide on the healing of skin ulcers in db/db mice

**Experimental animals:** Diabetic model mice (db/db male mice), normal mice (m/m male mice), SPF grade, 8-10 weeks of age, weighting 40-50 g, from Changzhou Cavens Experimental Animal Co., Ltd., with the experimental animal production license number of SCXK (Su) 2016-0010, and the experimental animal use license number of SYXK (Shanghai) 2020-0038.

**Experimental method:** After the blood glucose of the db / db mice reached the standard (blood glucose after fasting for 4h ≥ 16.7 mmol/L), the mice were randomly divided into 5 groups with 10 mice in each group, namely, normal control group, model control group, Kangfuxin Ye group (40 µL per mouse), GeneTime group (40 IU/cm²), the subject polypeptide group (30 µg /cm²). After the mice were anesthetized with isoflurane inhalation, a circular wound with a diameter of about 12 mm was cut on the back of the mice with a cutter from the full layers of the skin to reach the fascia layer. The wound was photographed and the wound area was recorded as the baseline value (the wound was recorded as DayO). From the next day (Day 1), the animals in each group were administered as required, and all the skin wounds were applied externally. Both the normal control group and the model control group were given 40 µL of normal saline, and the GeneTime group (40 IU/cm²) was given Recombinant human epidermal growth factor topical solution diluted in 40 µL of normal saline (Shenzhen Watsin Genetech Ltd.), and the subject polypeptide group (30 µg/cm²) was given the corresponding concentration of the polypeptide prepared by using 40 µL normal saline, once a day; the Kangfuxin Ye group was given 40 µL of Kangfuxin Ye (SICHUAN GOODDOCTOR PANXI PHARMACEUTICAL CO., LTD.), twice a day. Administration was continued every day until the wound was basically healed. The skin wounds were measured and photographed twice a week, the areas of the wounds were calculated with the Image-J software, and the healing of the wounds in each group of animals was observed.

**Experimental results:** The experimental results are shown in Table 4:

**Table 4 Wound healing rate (%, Mean±SD, n =10)**

| Group | Day0 | Day3 | Day7 | Day10 | Day14 |
|---|---|---|---|---|---|
| Normal control group (40 µL/mouse, qd) | 0.00±0.00 | -12.19±10.62 | 27.28±9.36 | 56.22±14.38 | 96.11±4.04 |
| Model control group (40 µL/mouse,qd) | 0.00±0.00 | -10.04±12.76 | 0.89±14.75 | 31.1±12.52 | 56.57±11.39 |
| Kangfuxin Ye group (40 µL/mouse, Bid) | 0.00±0.00 | 0.48±17.51 | 15.37±9.41* | 37.24±6.79 | 82.63±8.87** |
| GeneTime group (40 IU/cm², qd) | 0.00±0.00 | -2.21±12.34 | 19.07±10.00** | 44.33±10.23* | 92.23±5.73** |
| The polypeptide group (30 µg/cm², qd) | 0.00±0.00 | -4.66±17.55 | 13.06±17.65 | 40.91±16.33 | 89.44±8.99** |

| | | | | | |
|---|---|---|---|---|---|
| Note: *: *P* <0.05; **: *P* <0.01 compared with the model control group; | | | | | |

The research results in Table 4 show that the polypeptide can significantly promote skin wound healing in diabetic mice on day 14, and the healing effect was better than that of the Kangfuxin Ye group, and was comparable to that of the GeneTime group.

### Example 3 Healing effect of the polypeptide on acute mechanical skin injuries in rats

**Experimental animals:** SD male rats, SPF grade, 6 weeks old, weighing 180-220 g, from Beijing Vital River Laboratory Animal Technology Co., Ltd.; animal license number: SCXK (Beijing) 2016-0006; and certification number: NO.110011210104746538.

**Experimental method:** SPF grade SD rats were housed in clean and sterilized cages, and were given water and feeds and padding replacement at regular intervals every day. The cultivation was kept at a temperature of 20-26°C with a humidity of 40%-70%. The rats were kept for one week to adapt to the environment. According to the random grouping method, the animals were divided into 3 groups: model control group (normal saline), GeneTime group (40 IU/cm², recombinant human epidermal growth factor topical solution, from Shenzhen Watsin Genetech Ltd.), and subject polypeptide group (8 µg/cm²), with 6 animals in each group. After the rats were successfully anesthetized by intraperitoneal injection of 3% pentobarbital sodium, the hair at 1 cm from the expected wound edge was cut off, the expected wound area was first disinfected with iodophor, then locally disinfected with 75% alcohol, and a round full layer of skin wound with a diameter of 1.5 cm was made at the middle of the ear line down to 4 cm below the back, near the cervical side, with the spine as the middle line, as deep as the muscular layer. The same sized rubber ring was used to fix the surrounding skin to form an animal model of acute mechanical injury. After modeling, the rats' wounds were exposed to the environment and fed in individual cages. Upon changing dressings, debridement was first performed with iodophor, and then the wound was washed with a sterile normal saline and wiped to dryness. Rats in each group were given 40 µL of corresponding drug solution by locally applying to the wound once a day periodically. On Days 0, 3, 7, 10 and 14 of administration, the wounds of each group of rats were photographed, the areas of the wounds were calculated with image analysis software (Image J), and the wound healing rates were calculated according to the formula.

**Experimental results:** The experimental results are shown in Table 5:

**Table 5 Wound healing rate (%, Mean ± SD, n = 6**

| Group | Day0 | Day3 | Day7 | Day10 | Day14 |
|---|---|---|---|---|---|
| Model control group | 0.00±0.00 | -9.93±8.77 | -23.22±3.44 | 11.64±9.81 | 67.59±8.26 |
| GeneTime group | 0.00±0.00 | -15.09±5.68 | -11.45±7.33 | 21.45±9.59 | 63.29±6.03 |
| The Polypeptide group (8 µg/cm²) | 0.00±0.00 | -23.77±6.16 | -10.78±7.90 | 18.90±17.63 | 88.42±4.57** |

| | | | | | |
|---|---|---|---|---|---|
| Note: *: *P* < 0.05, **: *P* < 0.01 compared with the model control group; | | | | | |

The research results of Table 5 show that the polypeptide group on Day 14 showed significant promotion of wound healing after mechanical skin injury in rats, and the effect was significantly better than that of the GeneTime group.

### Example 4 Proliferation-promoting effect of the polypeptide on HaCAT cells

**Experimental method:** Human immortalized keratinocytes (HaCAT cells) were adjusted to a concentration of 1.0 * 10⁵ to 5.0 * 10⁵/mL for passaging, and cultured at 37°C and 5% CO₂ for 24-36 hours for the biological activity detection. The cells were digested with 0.25% pancreatin for 5 min, a 1640 whole blood medium with more than 1 times the volume of pancreatin was added to stop digestion, a cell suspension was collected and centrifuged at 1000 RPM for 3 min, the supernatant was discarded, 2 mL of 1640 whole blood medium was added to resuspend the cells, 20 uL cell suspension was taken, stained with AOPI, and then the concentration of cells in the suspension was detected with a cell counter, the cell suspension was formulated with 1640 medium with 10% serum to a concentration of 5 * 10⁴/mL and seeded into 96-well cell culture plates with 100 µL per well, i. e. 5000 cells/well, and cultured overnight at 37°C and 5% CO₂.

After 24 hours, the original culture medium was discarded and 100 µL of each of different concentrations of polypeptide solutions formulated with 1640 culture medium with 1% serum was added. At the same time, EGF control group was set, i.e. 100 µL of recombinant human epidermal growth factor (EGF) solution formulated with 1640 medium with 1% serum was added, with a final concentration of 100 ng/mL. For the model control group, 1640 medium with 1% serum of equal volume was added. The mixtures were cultured at 37°C and 5% CO₂ for 72 h, and the proliferation of the HaCaT cell line was detected by the CCK8 kit.

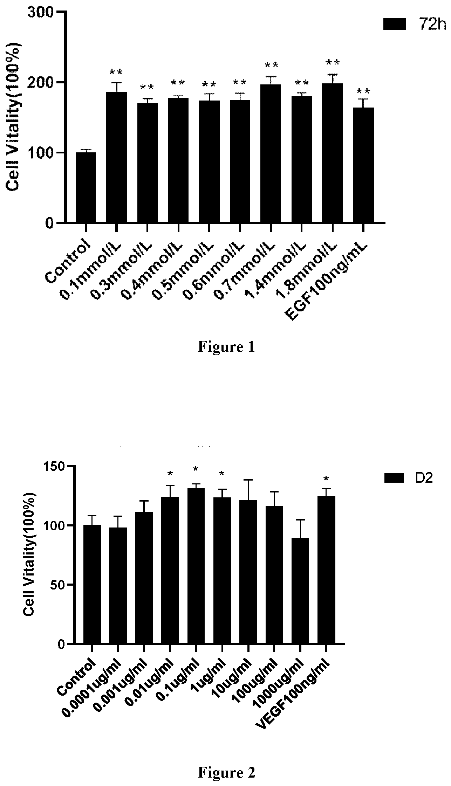

**Experimental results:** The experimental results are shown in Figure 1 (*: *P* < 0.05; **: *P* < 0.01). The research results of Figure 1 show that the polypeptide has the significant effect of promoting proliferation of epidermal cells as one type of the main cells involved in the wound healing process, i.e. HaCaT cells (human immortalized keratinocytes), after 72 hours of action.

### Example 5 Proliferation-promoting effect of the polypeptide on HMEC-1 cells

**Experimental method:** Human microvascular endothelial cells (HMEC-1 cells) were cultured in 10% serum medium at 37°C and 5% CO₂, the culture fluid was changed every 1-2 days, and the cell concentration was controlled to be 4 * 10⁶cells/mL for passaging. The cells were collected and prepared into a concentration of 4 * 10⁴cells/mL with a serum-free medium, seeded into a 96-well cell culture plate with 100 uL per well, i.e., 4000 cells/well, and cultured at 37°C and 5% CO₂ to be adherent.

The polypeptide was dissolved in PBS solution and prepared into a stock solution with a concentration of 1 mg/mL. The polypeptide stock solution was prepared with 0% serum medium to test concentrations as the experimental groups; in the same manner, recombinant human vascular endothelial growth factor VEGF (100 ng/mL) was prepared and added as positive control; and for the blank control group, the same volume of 0% serum medium without drugs was added. For all the groups, 5 duplicate wells were incubated at 37°C and 5% CO₂ for 48 hours. The CCK-8 was used to detect cell proliferation: serum-free medium containing 10% CCK-8 was added to each well, and absorbances were measured at 450 nm by microplate reader after incubation in an incubator for 2 hours.

**Experimental results:** The experimental results are shown in Figure 2 (*: *P* < 0.05; **: *P* < 0.01). The research results of Figure 2 show that the polypeptide has the significant effect of promoting proliferation of endothelial cells as one type of the main cells involved in the wound healing process, i.e. HMEC-1 cells (human microvascular endothelial cells).

### Example 6 Proliferation-promoting effect of the polypeptide on Balb-3T3 cells

**Experimental method:** Mouse embryo fibroblasts (Balb-3T3 cells) were cultured in 10% serum medium at 37°C and 5% CO₂, the culture fluid was changed every 1-2 days, and the cell concentration was controlled to be 4 * 10⁶cells/mL for passaging. The cells were collected and prepared into a concentration of 3 * 10⁴cells/mL with 2.5% FBS maintenance medium, seeded into a 96-well cell culture plate with 100 uL per well, i.e., 3000 cells/well, and cultured at 37°C and 5% CO₂ to be adherent.

The polypeptide was dissolved in 2.5% FBS maintenance medium and prepared into a stock solution with a concentration of 1 mg/mL. The polypeptide stock solution was prepared with 2.5% FBS maintenance medium to test concentrations as the experimental groups; in the same manner, recombinant human basic fibroblast growth factor FGF (50 ng/mL), and recombinant human platelet derived growth factor PDGF-BB (30 ng/mL) were prepared and added as positive control; and for the blank control group, the same volume of 2.5% FBS maintenance medium was added. For all the groups, 5 duplicate wells were incubated at 37°C and 5% CO₂ for 48 hours. CCK-8 was used to detect cell proliferation: the serum-free medium containing 10% CCK-8 was added to each well, and absorbances were measured at 450 nm by microplate reader after incubation in an incubator for 2 hours.

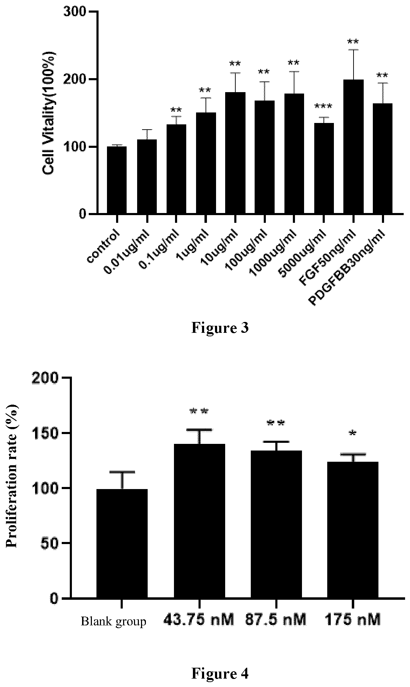

**Experimental results:** The experimental results are shown in Figure 3 (*: *P* < 0.05; **: *P* < 0.01). The research results of Figure 3 show that the polypeptide has the significant effect of promoting proliferation of fibroblasts as one type of the main cells involved in the wound healing process, i.e. Balb-3T3 cells (mouse embryo fibroblasts).

### Example 7 The effect of the polypeptide on RSC96 cell proliferation

**Experimental method:** Rat Schwann cells (RSC96 cells) were adjusted to a concentration of 1.0 * 10⁵ to 5.0 * 10⁵/mL for passaging, and cultured at 37°C and 5% CO₂ for 24-36 hours for the biological activity detection. The cells were digested by trypsin, collected, prepared into a concentration of 5 * 10⁴/mL with a serum-free medium, seeded in a 96-well cell culture plate with 100 µL per well, i.e., 8000 cells/well, and cultured overnight at 37°C and 5% CO₂.

The polypeptide was dissolved in PBS solution and prepared into a stock solution with a concentration of 400 ug/ml. The polypeptide stock solution was prepared with 0% serum medium to test concentrations as the experimental groups; and for the control group, the same volume of 0% serum medium without drugs was added. For all the groups, 4 duplicate wells were incubated at 37°C and 5% CO₂ for 48 hours. The CCK-8 was used to detect cell proliferation: the old medium was removed, the serum-free medium containing 10% CCK-8 was added to each well, and absorbances were measured at 450 nm by microplate reader after incubation in an incubator for 2 hours.

**Experimental results:** The experimental results are shown in Figure 4 (*: *P* < 0.05; **: *P* < 0.01). The research results of Figure 4 show that the polypeptide has the significant effect of promoting proliferation of RSC96 cells.

### Example 8 The effect of the polypeptide in promoting tissue regeneration in zebrafish

**Experimental animals:** Wild-type AB-line zebrafish, raised in water for culturing fish at 28°C (water quality: 200 mg of instant dissolving sea salt being added to every 1 L of water made by reverse osmosis, with a conductivity of 450-550 µS/cm, a pH of 6.5-8.5 and a hardness of 50-100 mg/L CaCO₃), at the age of 3 days post fertilization (3 dpf). The animals were provided by the fish breeding center of Hangzhou Hunter Biotechnology Co., Ltd. with the experimental animal use license number of SYXK (Zhejiang) 2012-0171, and the breeding management complied with the requirements of international AAALAC certification (certification number: 001458).

**Experimental method:** 3 dpf wild AB-line zebrafish were randomly selected, and the caudal fins of zebrafish was excised to establish a model of caudal fin damage in zebrafish. The model zebrafish were randomly assigned to a 6-well plate, with 30 zebrafish per well (per experimental group). Different concentrations of polypeptides (with final concentrations of 250, 500 and 1000 µg/mL) were given in aqueous solutions respectively, while normal control group (without damages in caudal fins of zebrafish) and model control group were set with a volume of 3 mL per well. Treatment was performed at 28°C for 3 days, 10 zebrafish were randomly selected in each experimental group and placed under an anatomical microscope to take photos, the data were analyzed and collected using NIS-Elements D 3.20 advanced image processing software, the regeneration areas of caudal fins of zebrafish were analyzed, and the statistical analysis results of this index were used to evaluate the efficacy of the samples of promoting tissue regeneration.

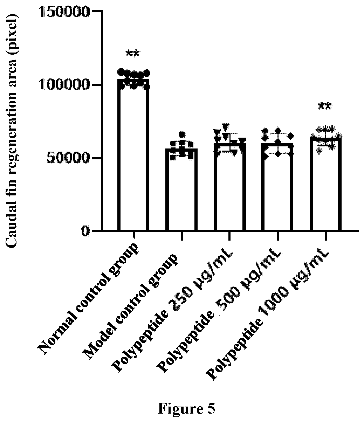

**Experimental results:** The experimental results are shown in Figure 5 (*: *P* < 0.05, **: *P* < 0.01 compared with the model control group). The research results show that the polypeptide has the efficacy of significantly promoting tissue regeneration in zebrafish.

### Example 9 The effect of the polypeptide in promoting angiogenesis in zebrafish

**Experimental animals:** Transgenic zebrafish with vascular green fluorescence, raised in water for culturing fish at 28°C (water quality: 200 mg of instant dissolving sea salt being added to every 1 L of water made by reverse osmosis, with a conductivity of 450-550 µS/cm, a pH of 6.5-8.5 and a hardness of 50-100 mg/L CaCO₃), at the age of 1 day post fertilization (1 dpf). The animals were provided by the fish breeding center of Hangzhou Hunter Biotechnology Co., Ltd. with the experimental animal use license number of SYXK (Zhejiang) 2012-0171, and the breeding management complied with the requirements of international AAALAC certification (certification number: 001458).

**Experimental method:** 1 dpf transgenic zebrafish with vascular green fluorescence were randomly selected and placed in a 6-well plate, with 30 fish per well (per experimental group). The zebrafish in the normal control group were treated with standard dilution water, and each of the other experimental groups was induced with 60 nM of aqueous simvastatin for 3 hours to establish a model of zebrafish with microvessel deficiency, with a volume of 3 mL per well. After 3 h, simvastatin induction was terminated. Then the aqueous solution of the positive control group was replaced with 5.90 µg/mL astragaloside IV, and the aqueous solution of each of the other groups was replaced with standard dilution water, with a volume of 3 mL per well. The test drug groups were given different doses of the polypeptide via intravenous injection, i.e. with the concentrations of 12.5, 25.0 and 50.0 mg/mL respectively, and the injection volume of 10 nL. Treatment was performed at 28°C for 2 days, 10 zebrafish were randomly selected in each experimental group and placed under a fluorescence microscope to take photos, the data were analyzed and collected using NIS-Elements D 3.20 advanced image processing software, the subintestinal vascular area and the number of subintestinal vascular branches were analyzed, and the statistical analysis results of these indexes were used to evaluate the efficacy of the samples of promoting angiogenesis.

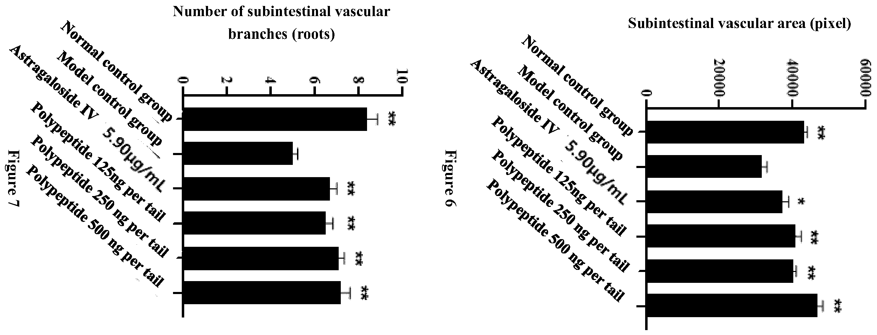

**Experimental results:** The experimental results are shown in Figure 6 and Figure 7 (*: *P* < 0.05, **: *P* < 0.01 compared with the model control group). The research results show that the polypeptide can significantly promote the increase of the subintestinal vascular area and the increase of the number of subintestinal vascular branches in zebrafish, and has the efficacy of promoting angiogenesis.

### Example 10 The healing effect of the polypeptide on skin ulcer induced by streptozotocin (STZ) in diabetic rats

**Experimental animals:** After SPF grade male healthy SD rats (weighing 180-200 g) were fed with high-fat and high-sugar diet for 2 weeks and fasted for 6 hours, each rat was rapidly injected with an STZ solution (50 mg kg⁻¹) intraperitoneally at one time, and 48 hours later, the random blood glucose values were > 16.7 mmol/L twice, indicating the successful establishment of type II diabetic rat model. Experimental animals were purchased from Beijing Vital River Laboratory Animal Technology Co., Ltd., with the experimental animal quality certification number of SCXK (Beijing) 2017-0033, and the experimental animal use license number of SCXK (Beijing) 2017-0033.

**Experimental method:** 90 diabetic rats were selected and randomly divided into 6 groups with 15 rats in each group, namely, model control group (normal saline), high-dose polypeptide group (30 µg/cm²), medium-dose polypeptide group (10 µg/cm²). and low-dose polypeptide group (3 µg/cm²), GeneTime group (40 IU/cm², recombinant human epidermal growth factor topical solution, from Shenzhen Watsin Genetech Ltd.), and Kangfuxin Ye group (36 µL/cm², Kangfuxinye, from SICHUAN GOODDOCTOR PANXI PHARMACEUTICAL CO., LTD.). After each experimental rat was anesthetized by intraperitoneal injection of 2% pentobarbital sodium (0.2 mL/100 g), a 2 cm² full-layer skin defect wound was prepared at each of both sides of the back spine, and a rubber ring with a diameter of 2.3 cm was stitched at the outer edge of the wound. After hemostasis of the wound, local drug therapy at the wound was carried out by group. Except that the Kangfuxin Ye group was administered 2 times/day, other groups were administered once a day, with a dosed volume of 72 µL/wound each time, for 14 consecutive days. After the administration, the wound was covered with a sterile vaseline gauze (5 cm * 5 cm), and bandaged with multi-layer sterile gauzes. After the wound surgery, the wound was photographed, and the wound area was recorded as the baseline value (the wound was recorded as DayO). During the administration period, the wound areas were photographed and measured 3 times a week, and the granulation tissue growth and healing of wound in each group were observed every day.

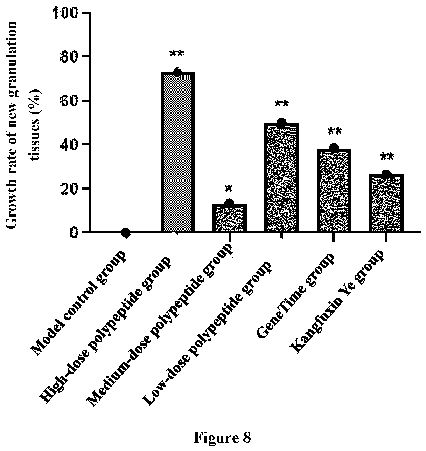

**Experimental results:** See Table 6 and Figure 8 (Note: *: *P* < 0.05; **: *P* < 0.01 compared with the model control group).

As can be seen from Table 6, on Day 10, the healing rates of the low-dose and medium-dose polypeptide groups were slightly higher than the healing rate of the model control group, indicating that the polypeptide at low and medium doses had a tendency to promote the wound healing in diabetic rats; in the whole process of wound healing, the healing rate of the high-dose polypeptide group was higher, and was significantly higher than the model control group and slightly higher than the GeneTime group on Day 10, and slightly higher than that of the Kangfuxin Ye group on Days 7-12, indicating that the high-dose polypeptide group had a significant healing promoting effect on the wound of diabetic rats, and the effect was slightly better than that of the GeneTime in this experiment. As can be seen from Figure 8, the low-, medium- and high-dose polypeptide groups promoted the growth rate of new granulation tissue compared to the model control group with significant differences. Therefore, the polypeptide has the effect of promoting the growth of new granulation tissue at the wound and wound healing in STZ-induced diabetic rats.

**Table 6 Wound healing rates (%, Mean ± SD, n = 15) of diabetic rats at different days post injury in each group**

| Group | Day0 | Day3 | Day7 | Day 10 | Day 12 | Day 14 |
|---|---|---|---|---|---|---|
| Model control group | 0.00±0.00 | 1.93±6.99 | 57.00±13.24 | 80.94±5.43 | 96.80±3.44 | 99.84±0.89 |
| High-dose polypeptide group | 0.00±0.00 | 6.82±19.81 | 62.62±12.24 | 88.49±5.17** | 98.39±2.39 | 99.85±0.77 |
| Medium-dose polypeptide group | 0.00±0.00 | 5.04±9.31 | 57.57±10.49 | 84.26±9.10 | 97.77±3.71 | 99.85±0.84 |
| Low-dose polypeptide group | 0.00±0.00 | 3.05±8.10 | 54.67±18.10 | 84.05±10.21 | 95.92±5.82 | 99.46±1.46 |
| GeneTime group | 0.00±0.00 | 4.71±6.20 | 60.53±8.87 | 87.61±5.68* | 98.64±2.28 | 100.00±0.00 |
| Kangfuxin Ye group | 0.00±0.00 | 7.59±8.10* | 61.08±14.30 | 86.75±7.59** | 97.80±5.01 | 100.00±0.00 |

### Example 11 Healing effect of the polypeptide on full-layer skin defect wound in SD rats

**Experimental animals:** SD male rats, SPF grade, 6 weeks old, weighing 180-220 g, from Beijing Vital River Laboratory Animal Technology Co., Ltd., with the experimental animal quality certification number of NO.110011220109610345, and the experimental animal use license number of SCXK (Beijing) 2021-0011.

**Experimental method:** SD rats were randomly divided into 8 groups based on the body weight with 8 animals in each group, namely, vehicle group, the polypeptide group (50 µg/cm²), comparative polypeptide 1 group (41.90 µg/cm²) and comparative polypeptide 2 group (52.66 µg/cm²). The amino acid sequence of comparative polypeptide 1 is Glu-Pro-Val-Pro-Leu; and the amino acid sequence of comparative polypeptide 2 is Pro-Ala-Ala-Glu-Pro-Val-Pro-Leu- Val-Lys-Gln-Glu.

After each experimental rat was anesthetized by intraperitoneal injection of 2% pentobarbital sodium (0.2 mL/100 g), a 2 cm² full-layer skin defect wound was prepared at each of both sides of the back spine. After hemostasis of the wound, the corresponding drug was administered at the wound locally by group. Each group was administered once a day, with a dosed volume of 72 µL/wound each time, for 14 consecutive days. After the administration, the wound was covered with a sterile vaseline gauze (5 cm * 5 cm), and bandaged with multi-layer sterile gauzes. After the wound surgery, the wound was photographed, and the wound area was recorded as the baseline value (the wound was recorded as DayO). During the administration period, the areas of the wounds were photographed and measured 3 times a week, and the healing was observed.

**Experimental results:** The experimental results are shown in Table 7:

**Table 7 Wound healing rate (%, Mean ± SD, n = 8)**

| | Day0 | Day3 | Day 5 | Day7 | Day 10 |
|---|---|---|---|---|---|
| Vehicle group | 0.00±0.00 | 11.01±23.19 | 38.94±18.95 | 61.81±11.97 | 87.77±5.65 |
| Polypeptide group | 0.00±0.00 | 29.70±19.21* | 52.21±11.54* | 74.12±5.95*** | 94.55±2.40*** |
| Comparative polypeptide 1 group | 0.00±0.00 | 21.13±17.43 | 40.18±17.43 | 68.84±7.64 | 88.90±8.40 |
| Comparative polypeptide 2 group | 0.00±0.00 | 17.21±22.78 | 50.08±16.55 | 65.26±14.46 | 90.34±3.04 |

| | | | | | |
|---|---|---|---|---|---|
| Note: *: *P* < 0.05; **: *P* < 0.01 compared with the vehicle group; | | | | | |

The research results show that, compared with the vehicle group, the polypeptide group of the present invention (50 µg/cm²) had a higher healing rate with a significant difference on days 3-10 after surgery, and the healing effect of the polypeptide of the present invention was better than that of the comparative polypeptide 1 group and the comparative polypeptide 2 group.

In summary, compared with the recombinant human epidermal growth factors commonly used in the treatment of skin injury diseases in the art, the polypeptide provided herein not only has obvious wound-healing effect on skin ulcers and injuries, especially acute and/or chronic skin diseases, diabetic foot ulcers and chronic refractory wounds on a body surface such as diabetic foot ulcers, pressure ulcers, vascular ulcers and infectious ulcers, but also can be absorbed by the skin much faster and much better due to its short chain of the peptide so as to have good stability in vivo and in vitro, and have a significant pro-proliferation effect on human immortalized keratinocytes, human microvascular endothelial cells, fibroblasts, glial cells, and tissue regeneration and angiogenesis. The polypeptide of the present invention is suitable for preparing products for preventing or treating skin injury diseases, and can produce positive therapeutic and repair effects.

Although the above examples are disclosed in the present invention, the embodiments of the present invention are not limited to the above examples, and any other changes, modifications, substitutions, combinations, and simplifications that do not depart from the present invention should be equivalent replacements and are included in the scope of protection of the present invention.

## Claims

1. A use of a polypeptide in the preparation of a product for preventing or treating skin injury diseases, the polypeptide is Pro-Ala-Ala-Glu-Pro-Val-Pro-Leu or a physiologically compatible salt thereof.\

2. The use of claim 1, wherein the skin injury diseases comprise wounds, ulcers, dermatitis, eczema, urticaria, polymorphic light rash, herpes, acne, impetigo, chloasma, vitiligo, lupus erythematosus, dermatomyositis, scleroderma, folliculitis, scabies, onychomycosis, chickenpox, roseola infantilis, warts, carbuncles, boils or ringworms.

3. The use of claim 2, wherein the dermatitis comprises atopic dermatitis, contact dermatitis, neurodermatitis, seborrheic dermatitis, hormone-dependent dermatitis or stasis dermatitis.

4. The use of claim 2, wherein the wounds comprise chronic refractory wounds on a body surface.

5. The use of claim 4, wherein the chronic refractory wounds on a body surface comprise diabetic foot ulcers, pressure ulcers, vascular ulcers and infectious ulcers.

6. The use of claim 1, wherein the product comprises medicines, skin care products or cosmetics.

7. The use of claim 1, wherein the product is a topical preparation.

8. The use of claim 7, wherein the topical preparation comprises a solution, emulsion, gel, cream, spray, mask or dressing.

9. The use of claim 7, wherein the polypeptide repairs or heals the wounds by promoting the proliferation of human immortalized keratinocytes, human microvascular endothelial cells, fibroblasts, glial cells, and tissue regeneration and/or angiogenesis.
